Europäisches Patentamt

European Patent Office

Office européen des brevets

(11) Veröffentlichungsnummer: **0 499 903 A1**

# EUROPÄISCHE PATENTANMELDUNG

(21) Anmeldenummer: **92102047.5**

(51) Int. Cl.5: **A61M 5/172**

(22) Anmeldetag: **07.02.92**

(30) Priorität: **16.02.91 DE 4104814**

(43) Veröffentlichungstag der Anmeldung:
**26.08.92 Patentblatt 92/35**

(84) Benannte Vertragsstaaten:
**AT BE CH DE DK ES FR GB IT LI NL PT SE**

(71) Anmelder: **B. Braun Melsungen AG**
**Carl-Braun Strasse**
**W-3508 Melsungen(DE)**

(72) Erfinder: **Knoche, Alfred, Dipl.-Ing.**
**Herkulesstrasse 8**
**W-3501 Körle(DE)**
Erfinder: **Mosebach, Wolfgang**
**Hügelskopf 7**
**W-3509 Dagobertshausen(DE)**
Erfinder: **Heitmeier, Rolf, Dipl.-Ing.**
**Goethestrasse 8**
**W-3507 Baunatal(DE)**

(74) Vertreter: **Selting, Günther, Dipl.-Ing. et al**
**Patentanwälte von Kreisler, Selting, Werner**
**Deichmannhaus am Hauptbahnhof**
**W-5000 Köln 1(DE)**

(54) **Druckinfusionsapparat.**

(57) Der Druckinfusionsapparat weist ein Steuergerät auf, an dem durch Eingabe an einem Bedienfeld (19) unterschiedliche Betriebsarten eingestellt werden können, wie z.B. Dauerinfusion oder Bolusbetrieb. Zur Bestimmung der auszuführenden Betriebsart dient ein externer Datenträger (30), der in das Gehäuse (13) eingeführt werden kann und eine Kodierung für die vorgesehene Betriebsart trägt. Der Datenträger (30) ist mit einer von außerhalb des Gehäuses gut sichtbaren Erkennungseinrichtung (35) versehen. Der Druckinfusionsapparat kann als Grundmodell ausgeführt werden, das zahlreiche Betriebsarten wählbar ausführen kann. Die jeweils gewählte Betriebsart ist durch die zugehörige Erkennungseinrichtung (35) auch bei abgeschaltetem Apparat gut sichtbar. Es wird eine optische Individualisierung der Druckinfusionsapparate nach Betriebsarten ermöglicht.

FIG.1

EP 0 499 903 A1

Die Erfindung betrifft einen Druckinfusionsapparat der im Oberbegriff des Patentanspruchs 1 angegebenen Art.

Druckinfusionsapparate werden dazu benutzt, Patienten eine Infusionsflüssigkeit mit vorbestimmbarer wählbarer Rate aus einem Vorratsbehälter zuzuführen. Wenn der Vorratsbehälter eine Flasche o.dgl. ist, wird die Flüssigkeit in der Regel durch Schwerkrafteinfluß dem Vorratsbehälter mit einer Tropfvorrichtung entnommen und über einen Schlauch dem Patienten zugeführt. Der Schlauch führt durch eine peristaltische Schlauchpumpe hindurch, die die Flüssigkeit mit vorbestimmter Rate fördert. Wenn der Vorratsbehälter eine Spritze ist, besteht der Druckinfusionsapparat aus einer Spritzenpumpe mit einem Schieber, der den Spritzenkolben im Spritzenzylinder mit vorbestimmter Rate vorschiebt.

Die erläuterten Druckinfusionsapparate sind für unterschiedliche Betriebsarten ausgelegt. So gibt es Druckinfusionsapparate, die eine kontinuierliche Förderung bewirken, und Druckinfusionsapparate, die für den Bolusbetrieb bestimmt sind, bei dem jeweils innerhalb einer Boluszeit eine bestimmte Förderrate aufrechterhalten wird, wonach eine einstellbare Pausenzeit folgt. Ferner gibt es patientengesteuerte Druckinfusionsapparate, bei denen der Patient auf Knopfdruck die Verabreichung eines Bolus wählen kann. Druckinfusionsapparate können auch in Verbindung mit einem Durchflußmesser betrieben werden, der die tatsächliche Durchflußrate mißt und die Pumpengeschwindigkeit so regelt, daß die eingestellte Förderrate aufrechterhalten wird.

Diese unterschiedlichen Betriebsarten, die bei Druckinfusionsapparaten möglich sind, erfordern in der Praxis unterschiedliche Typen von Druckinfusionsapparaten. Zwar ist es generell möglich, die Steuereinrichtung eines Druckinfusionsapparates so zu konzipieren, daß das Gerät jeweils wahlweise eine von mehreren Betriebsarten ausführen kann, jedoch ist dies aus Gründen der Handhabung und Betriebssicherheit nicht zweckmäßig. Kliniken verfügen oft über Hunderte von Druckinfusionsapparaten und das Klinikpersonal ist daran gewöhnt, jeweils für eine bestimmte Betriebsart einen anderen Apparatetyp einzusetzen. Hätten alle Apparate das gleiche Aussehen und würden sie sich hinsichtlich ihrer Betriebsarten nur durch eine zuvor vorgenommene Programmierung unterscheiden, dann würde die Gefahr bestehen, daß für einzelne Anwendungsfälle das falsche Gerät benutzt wird. Selbst wenn die für den betreffenden Apparat einprogrammierte Betriebsart an einer Anzeigevorrichtung angezeigt wird, besteht die Gefahr, daß die Anzeige übersehen wird. Nachteilig ist ferner, daß die Anzeige nur bei eingeschaltetem Gerät in Funktion tritt, so daß die Betriebsart bei abgeschaltetem Gerät nicht erkennbar ist.

DE 27 58 368 C2 beschreibt einen Druckinfusionsapparat mit den Merkmalen des Oberbegriffs des Patentanspruchs 1. Bei diesem Druckinfusionsapparat sind ein externes Programmiergerät, eine Steuervorrichtung und eine Mikrodosiereinheit jeweils als separate Geräte vorgesehen, die vorübergehend miteinander verbunden werden können. Das Programmiergerät enthält eine Leseeinheit, mit der ein auf einem separaten Programmträger enthaltenes Programm des Tagesprofils einer Dosierung in einen internen Speicher eingelesen werden kann. Als Programmträger kommen Lochkarten, Lochstreifen oder Magnetkarten in Betracht. Das in das Programmiergerät einprogrammierte Dosierungsprogramm wird über ein vorübergehend installiertes Kabel in das Steuergerät übertragen, das die Mikrodosiereinheit steuert. Damit können zwar nicht unterschiedliche Betriebsarten gesteuert werden, jedoch unterschiedliche Zeitabläufe der Betriebsart "Bolusbetrieb", bei der Betrieb mit vorgegebener Bolusrate und Pausenzeiten einander abwechseln. Der Datenträger enthält sämtliche Programmdaten des bei dieser Betriebsart ablaufenden Steuerprogramms. Der Datenträger muß sämtliche für das jeweilige Steuerprogramm erforderliche Daten tragen und somit ein Detailprogramm enthalten. Weder an der Mikrodosiereinheit noch am Steuergerät ist erkennbar, welches Steuerprogramm geladen worden ist.

DE 24 51 424 beschreibt einen Druckinfusionsapparat, der aus einem Infusionsgerät und einem in einem separaten Gehäuse untergebrachten Programmgeber besteht. Der Programmgeber ist mit einem Kreuzschienenverteiler mit zahlreichen Steckbuchsen versehen, an dem der zeitliche Verlauf eines Infusionsprogramms durch Stecker programmiert werden kann.

Der Erfindung liegt die Aufgabe zugrunde, einen Druckinfusionsapparat zu schaffen, der in einer einzigen Bauart vielseitig verwendbar ist und dennoch entsprechend der jeweiligen Betriebsart vom äußeren Erscheinungsbild her individualisierbar ist.

Die Lösung dieser Aufgabe erfolgt mit den im Patentanspruch 1 angegebenen Merkmalen.

Der erfindungsgemäße Druckinfusionsapparat zeichnet sich dadurch aus, daß die Betriebsart mit einem externen Datenträger wählbar ist, der an dem Gehäuse angebracht bzw. in dieses eingeschoben wird und der eine Erkennungseinrichtung aufweist, die von außerhalb des Gehäuses gut sichtbar ist. Im Inneren des Gehäuses befindet sich eine Sensoreinrichtung, die die Daten des Datenträgers liest und daraufhin am Steuergerät die der gelesenen Kodierung entsprechende Betriebsart einstellt.

Der kodierte Datenträger bildet somit gewissermaßen einen Schlüssel für die mit ihm einzustellende Betriebsart. Das Gerät funktioniert in dieser Betriebsart nur dann, wenn es mit dem Datenträger versehen ist. In diesem Zustand ist die Erkennungseinrichtung des Datenträgers von außen deutlich sichtbar. Die Erkennungseinrichtungen von Datenträgern für unterschiedliche Betriebsarten können beispielsweise aus Platten von unterschiedlichen Farben bestehen, die zusätzlich mit der jeweiligen Betriebsart beschriftet sind. Eine solche Platte wird bei eingeschobenem Datenträger Bestandteil der Frontplatte des Gehäuses. Die Erkennungseinrichtung ermöglicht eine eindeutige Identifikation der Gerätevariante bzw. der Betriebsart auch im ausgeschalteten Zustand des Gerätes. Der Druckinfusionsapparat, der generell so konstruiert ist, daß er mehrere vorbestimmte, beispielsweise vorgespeicherte, Betriebsarten wahlweise ausführen kann, wird durch den Datenträger zu einem Druckinfusionsapparat, der nur für eine ganz bestimmte Betriebsart vorgesehen ist. Durch die am Datenträger angebrachte Erkennungseinrichtung wird das Gerätegehäuse als solches unverwechselbar für diese Betriebsart gekennzeichnet.

Die Erfindung bietet den Vorteil, daß ein Standard-Apparat durch die jeweiligen Datenträger für jeweils eine Betriebsart individualisiert werden kann, wobei der betreffende Datenträger gleichzeitig das Erscheinungsbild des Gehäuses mitbestimmt. Daher kann mit einem einzigen Grundgerät eine große Typenvielfalt realisiert werden. Von Vorteil ist ferner, daß die Erkennungseinrichtung den Apparatetyp auch dann optisch sichtbar kennzeichnet, wenn das Gerät abgeschaltet ist.

Die Erfindung eignet sich sowohl für Druckinfusionsapparate mit Schlauchpumpe als auch für Druckinfusionsapparate mit Spritzenpumpe. Der Datenträger ist vorzugsweise ein in das Gehäuse einschiebbarer Schieber, der eine Kodierleiste trägt und an seinem in Einschubrichtung rückwärtigen Ende die Erkennungseinrichtung in Form einer Platte aufweist.

Die Art der Kodierung des Datenträgers und die Art der Abtastung dieser Kodierung bieten zahlreiche Möglichkeiten. Die Kodierung kann beispielsweise geometrisch in Form von strukturellen Erhöhungen und Vertiefungen vorgesehen sein oder magnetisch in Form von magnetisierten Stellen des Datenträgers. Die Abtastung kann mit elektrischen Kontakten erfolgen, induktiv, optisch oder mit Mikroschaltern, die von Nocken oder Vorsprüngen des Datenträgers betätigt werden. Dabei können verschiedenartige Kodierungsarten benutzt werden, vorzugsweise solche mit zusätzlichen Prüfbits. Zweckmäßigerweise erfolgt die Kodierung im Binärsystem, weil hierdurch die Abtastung und Auswertung erleichtert wird, jedoch sind auch andere Systeme möglich.

Die Erfindung wird im folgenden unter Bezugnahme auf die Zeichnungen an einem Ausführungsbeispiel näher erläutert.

Es zeigen:

Fig. 1 eine perspektivische Ansicht des Druckinfusionsapparates,

Fig. 2 ein schematisches Blockschaltbild des Druckinfusionsapparates,

Fig. 3 eine Seitenansicht des Datenträgers einschließlich der Abtastvorrichtung und

Fig. 4 einen Schnitt entlang der Linie IV-IV von Fig. 3.

Der in den Zeichnungen dargestellte Druckinfusionsapparat 10 enthält eine Schlauchpumpe, die Infusionsflüssigkeit von einem Vorratsbehälter 11 durch einen Schlauch 12 hindurch zu einem Patienten fördert. Die Schlauchpumpe ist im Inneren des Gehäuses 13 des Druckinfusionsapparates angebracht. Dieses Gehäuse enthält in seiner Frontseite 14 einen Kanal 15, der durch eine aufklappbare Tür 16 verschlossen ist. Hinter der Tür 16 befindet sich die peristaltische Schlauchpumpe, die den Schlauch 12 fortlaufend abquetscht und dadurch den Vortrieb der Flüssigkeit bewirkt.

An dem dem Vorratsbehälter 11 zugewandten Ende des Schlauchs 12 ist eine Tropfvorrichtung 17 mit Einstechdorn befestigt. Der Einstechdorn wird in eine den Vorratsbehälter 11 verschließende Membran eingestochen, so daß Flüssigkeit aus dem Vorratsbehälter auslaufen kann. Der Einstechdorn enthält auch eine Belüftungseinrichtung, um das ausgelaufene Flüssigkeitsvolumen durch Luft zu ersetzen.

An der Tropfvorrichtung 17 ist eine Strömungsmeßeinrichtung 18 befestigt, die hier als Tropfenzähler ausgebildet ist, welcher die in der Tropfvorrichtung 17 herabfallenden Flüssigkeitstropfen zählt und ein entsprechendes Signal an den Druckinfusionsapparat 10 liefert. Anstelle des Tropfenzählers kann auch eine andere Strömungsmeßeinrichtung benutzt werden.

An der Frontseite 14 des Gehäuses 13 befindet sich ein Bedienfeld 19 mit einer alphanumerischen Anzeigevorrichtung 20 und verschiedenen Eingabe- und Bedientasten 21. In dem Bedienfeld 19 ist ferner ein Einführschlitz 22 vorgesehen, der sich hier über nahezu die gesamte Breite des Bedienfeldes erstreckt.

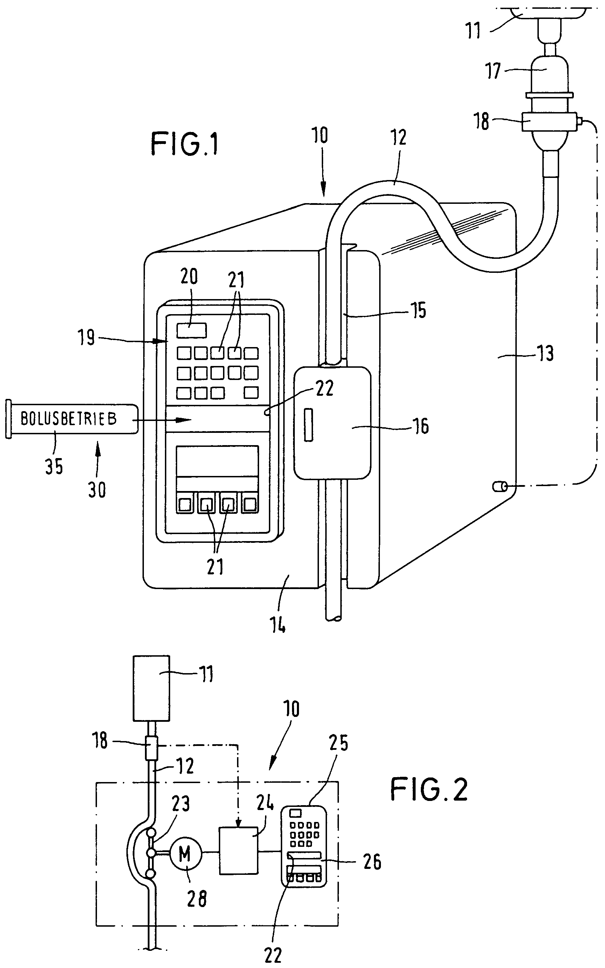

Aus Fig. 2 ist ersichtlich, daß die Flüssigkeit aus dem Vorratsbehälter 11 in den Schlauch 12 fließt, wobei die Strömungsrate von dem Durchflußmesser 18 überwacht wird. Der Schlauch 12 führt durch die Schlauchpumpe 23 hindurch und weiter zum Patienten. Der Strömungsmesser 18 kann sich auch in Strömungsrichtung hinter der Schlauchpumpe 23, also auf der Patientenseite des Schlauchs 12, befinden.

Der Rotor der Schlauchpumpe 23 wird von einem Motor 28 angetrieben, dessen Drehzahl von einer Steuereinrichtung 24 gesteuert wird. Die Steuereinrichtung 24 empfängt das Signal des Durchflußmessers 18 und sorgt dafür, daß die Förderrate einen vorbestimmten Wert einnimmt.

An die Steuereinrichtung 24 ist die Befehlseinrichtung 25 angeschlossen, zu der das Bedienfeld 26 gehört. Die Befehlseinrichtung 25 teilt der Steuereinrichtung 24 die Soll-Strömungsrate, die vorgesehene Betriebszeit und andere Infusionsparameter mit, sowie außerdem die vom Benutzer gewählte Betriebsart.

Als Betriebsarten sind beispielsweise möglich:
- Standardbetriebsart - kontinuierlicher Betrieb
- Bolusbetrieb - intermittierender Betrieb mit vorgegebener Bolusrate und Pausenzeit
- patientenkontrollierter Betrieb - der Patient kann über einen Taster die Zeitpunkte der Boluszuführung wählen
- Betrieb mit/ohne Durchflußüberwachung bei jeder der vorgenannten Betriebsarten.

Auf diese Weise können abhand der aufgezählten Möglichkeiten sechs verschiedene Betriebsarten eingestellt werden. Natürlich können auch noch weitere Betriebsarten möglich sein.

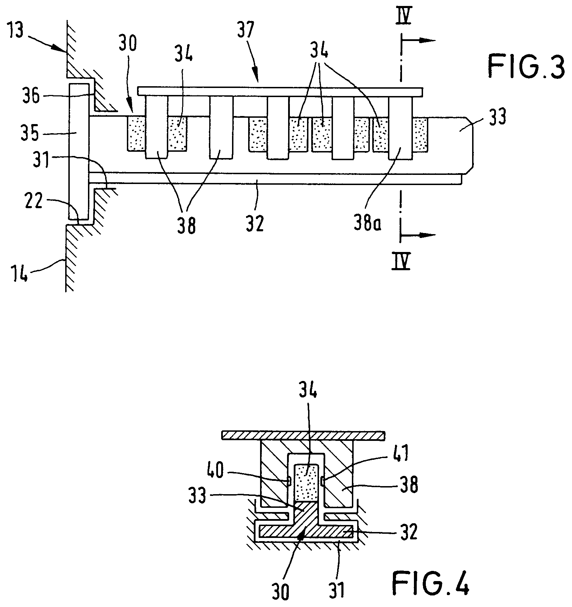

Die Wahl der jeweiligen Betriebsart erfolgt mit Hilfe eines separaten Datenträgers 30, der in den Einführschlitz 22 der Frontplatte 14 bzw. des Bedienfeldes 19 eingesteckt werden kann. Für jede Betriebsart steht ein anderer Datenträger 30 zur Verfügung. Jeder Datenträger ist als Schieber ausgebildet, der in einen hinter dem Einführschlitz 22 vorgesehenen Einschubkanal 31 gemäß Fig. 3 eingeschoben werden kann. Der starr ausgebildete Schieber weist eine horizontale Führungsplatte 32 und eine rechtwinklig davon aufragende Kodierleiste 33 auf. An der Kodierleiste 33 befinden sich Kodierstellen 34, die entsprechend der jeweiligen Kodierung als Vorsprünge oder Lücken ausgebildet sind. Bei dem vorliegenden Ausführungsbeispiel sind fünf Kodierstellen 34 vorhanden, von denen vier Kodierstellen Datenbits angeben, während die fünfte Kodierstelle ein Prüfbit angibt. Die vier Datenbits ermöglichen insgesamt sechszehn unterschiedliche Informationen (d.h. Betriebsarten).

An dem in Einschubrichtung rückwärtigen Ende des Datenträgers 30 ist eine Erkennungseinrichtung 35 in Form einer zur Kodierleiste 33 rechtwinkligen Platte 35 angebracht, die beim Einschieben des Datenträgers 30 gegen einen Anschlag 36 des Gehäuses stößt und die Einschubbewegung begrenzt. Die Erkennungseinrichtung 35 ist farbig gegenüber der Frontseite 14 bzw. dem Bedienfeld 19 abgesetzt, so daß das Vorhandensein des Datenträgers im Gehäuse 13 von außen her deutlich erkennbar ist. Die Datenträger 30 für unterschiedliche Betriebsarten unterscheiden sich durch unterschiedliche farbliche Gestaltungen der Erkennungseinrichtung 35 voneinander und zusätzlich durch die von außen sichtbaren Beschriftungen dieser Erkennungseinrichtungen.

Im Inneren des Gehäuses 13 ist am Einschubkanal 31 eine Sensoreinrichtung 37 vorgesehen, die eine der Anzahl der Kodierstellen entsprechende Anzahl von Sensoren 38 aufweist, von denen jeder eine der Kodierstellen 34 des eingeschobenen Datenträgers 30 abtastet. Der am weitesten im Gehäuseinnern angeordnete Sensor 38a erkennt, ob der Datenträger 30 vollständig eingeschoben ist bzw. ob die Erkennungseinrichtung 35 gegen den Anschlag 36 geschoben ist. Wenn der Sensor 38 die zugehörige Kodierstelle 34 erkannt hat, erfolgt das Lesen der Kodierung durch die Sensoreinrichtung 37 und die Weitergabe der entsprechenden Betriebsart-Information an das Steuergerät 24.

Bei dem vorliegenden Ausführungsbeispiel bestehen die Kodierungsstellen 34 aus Vorsprüngen oder Lücken. Die Sensoren 38 sind opto-elektrische Abtaster, die jeweils aus einer Lichtschranke bestehen. Jeder Sensor 38 weist einen umgekehrt-U-förmigen Träger auf, der an einem Schenkel mit einer Lichtquelle 40 und am anderen Schenkel mit einem Fotodetektor 41 versehen ist (Fig. 4). Wenn die Kodierstelle 34 ein Vorsprung ist, gelangt das Licht von der Lichtquelle 40 nicht zum Fotodetektor 41; ist dagegen die Kodierstelle 34 eine Lücke, so wird der Fotodetektor 41 von dem Licht der Lichtquelle 40 erregt.

Für den Druckinfusionsapparat 10 sind mehrere Datenträger 30 mit unterschiedlichen Kodierungen verfügbar. Der Benutzer wählt aus diesen Datenträgern denjenigen aus, der der gewünschten Betriebsart entspricht und steckt ihn einfach in den Einführschlitz 22 ein. Ein wesentlicher Vorteil besteht darin, daß die Betriebsart vom Benutzer gewählt werden kann, ohne daß der Benutzer komplizierte Einstellvorgänge ausführen muß. Die Einstellung der Infusionsparameter erfolgt in üblicher Weise an den Tasten 21 des Bedienfeldes 19.

Die Befehlseinrichtung 25 oder das Steuergerät 24 kann so ausgebildet sein, daß bei Nichtvorhandensein eines Datenträgers 30 eine Standard-Betriebsart eingestellt wird, beispielsweise kontinuierlicher Betrieb. Das Gerät kann jedoch auch so konzipiert sein, daß es ohne einen eingesteckten Datenträger 30 überhaupt nicht in Betrieb genommen werden kann.

Der Datenträger muß nicht notwendigerweise ein in das Gehäuse einschiebbarer Schieber sein sondern er kann beispielsweise auch eine steckbare Einrichtung sein, die von außen an das Gehäuse angesteckt wird, wobei die Sensoreinrichtung sich außen an der Gehäusewand befindet.

**Patentansprüche**

1. Druckinfusionsapparat mit

einer Pumpe (23), die Infusionsflüssigkeit von einem Vorratsbehälter (11) zu einem Patienten fördert,

einer in einem Gehäuse (13) untergebrachten Steuereinrichtung (24) zur Steuerung der Pumpe (23) nach einer von mehreren wählbaren Betriebsarten,

einem an dem Gehäuse (13) vorgesehenen Bedienfeld (19) zum Einstellen von Infusionsparametern, und

einer Sensoreinrichtung (37) zum Abtasten eines auswechselbaren Datenträgers (30),

**dadurch gekennzeichnet,**
daß die von der Sensoreinrichtung (37) gelesene Information des Datenträgers eine Kodierung ist, die angibt, nach welcher von mehreren vorbestimmten Betriebsarten die Steuereinrichtung (24) zu betreiben ist, und daß die Sensoreinrichtung (37) an oder in dem Gehäuse angeordnet und der Datenträger (30) mit einer während des Abtastens von außerhalb des Gehäuses gut sichtbaren Erkennungseinrichtung (35) versehen ist.

2. Druckinfusionsapparat nach Anspruch 1, dadurch gekennzeichnet, daß der Datenträger (30) in einen Einschubkanal (31) des Gehäuses (13) einschiebbar ist.

3. Druckinfusionsapparat nach Anspruch 2, dadurch gekennzeichnet, daß der Datenträger (30) ein Schieber ist, der an seinem in Einschubrichtung rückwärtigen Ende als Erkennungseinrichtung (35) eine Platte aufweist, die gegen einen gehäuseseitigen Anschlag (36) stößt.

4. Druckinfusionsapparat nach einem der Ansprüche 1 bis 3, dadurch gekennzeichnet, daß sich die Erkennungseinrichtung (35) bei am Gehäuse (13) angebrachtem Datenträger (30) an der das Bedienfeld (19) aufweisenden Frontseite (14) des Gehäuses befindet.

5. Druckinfusionsapparat nach einem der Ansprüche 1 bis 4, dadurch gekennzeichnet, daß der Datenträger (30) eine Kodierleiste (33) mit stationär abtastbaren Kodierstellen (34) aufweist.

6. Druckinfusionsapparat nach einem der Ansprüche 1 bis 5, dadurch gekennzeichnet, daß die Steuereinrichtung (24) ohne am Gehäuse (13) angebrachten Datenträger (30) eine Standard-Betriebsart ausführt.

7. Druckinfusionsapparat nach einem der Ansprüche 1 bis 5, dadurch gekennzeichnet, daß die Steuereinrichtung (24) ohne am Gehäuse (13) angebrachten Datenträger (30) funktionslos ist.

8. Druckinfusionsapparat nach einem der Ansprüche 1 bis 7, dadurch gekennzeichnet, daß die Pumpe (23) eine Schlauchpumpe ist.

9. Druckinfusionsapparat nach einem der Ansprüche 1 bis 8, dadurch gekennzeichnet, daß die Pumpe eine Spritzenpumpe ist.

FIG.1

BOLUSBETRIEB

FIG.2

FIG.3

FIG.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | CH-A-665 955 (UNIVERSO S.A.)<br>* Spalte 2, Zeile 9 - Zeile 14 *<br>* Spalte 2, Zeile 31 *<br>* Spalte 2, Zeile 48 - Zeile 66 *<br>* Spalte 3, Zeile 16 - Zeile 18 *<br>* Zusammenfassung; Anspruch 1; Abbildungen 1-5 * | 1,2 | A61M5/172 |
| Y | | 1,3,4,8 | |
| A | | 5,7 | |
| | --- | | |
| Y | EP-A-0 188 288 (INTELLIGENT MEDICINE, INC.)<br>* Seite 16, Zeile 6 - Zeile 15 *<br>* Seite 17, Zeile 20 - Zeile 24 *<br>* Seite 18, Zeile 12 - Zeile 19; Abbildung 4 * | 1 | |
| A | | 2,7,9 | |
| | --- | | |
| Y,D | EP-A-0 002 775 (SIEMENS A.G.)<br>* Seite 6, Zeile 32 - Zeile 34; Abbildung 1 *<br>* Seite 10, Zeile 31 - Zeile 33 *<br>* Seite 13, Zeile 29 - Zeile 35 * | 3,4,8 | |
| A,D | | 2 | |
| | ----- | | **RECHERCHIERTE SACHGEBIETE (Int. Cl.5)**<br><br>A61M |

Der vorliegende Recherchenbericht wurde für alle Patentansprüche erstellt

| Recherchenort | Abschlußdatum der Recherche | Prüfer |
|---|---|---|
| DEN HAAG | 25 MAI 1992 | SEDY R. |

EPO FORM 1503 03.82 (P0403)